# EUROPÄISCHE PATENTANMELDUNG

(11) **EP 0 806 217 A2**
(43) Veröffentlichungstag der Anmeldung: **12.11.1997**
(21) Anmeldenummer: 97106971.1
(22) Anmeldetag: 26.04.1997
(51) Int. Cl.: A61M 16/10, A61M 16/08

(54) **Beheizbarer Beatmungsschlauch und Verfahren zu seiner Herstellung**

(30) Priorität: 29.04.1996 DE 19617095
(71) Anmelder: WILLY RÜSCH AG, D-71394 Kernen-Rommelshausen (DE)
(72) Erfinder: Schmitt, Klaus, 73630 Remshalden-Grunbach (DE); Kostudis, Uwe, 01069 Dresden (DE); Weng, Horst, 73614 Schorndorf (DE); Singvogel, Armin, 71686 Remseck a.N. (DE)
(74) Vertreter: KOHLER SCHMID + PARTNER

(57) **Zusammenfassung**

Ein beheizbarer Beatmungsschlauch 10 besteht aus einem Schlauch 11 aus einem elastischen Material und einer daran angebrachten, in Längsrichtung des Schlauchs 11 verlaufenden Heizwendel 12 aus einem elektrisch leitfähigen Material. Die Außenumfangsfläche 13 der Heizwendel 12 ist an einer Innenoberfläche 14 im Lumen 15 des Schlauchs 11 befestigt. Daher kann die durch die Heizwendel 12 erzeugte Wärme effektiver auf die den Beatmungsschlauch 10 durchströmende Beatmungsluft übertragen werden. Gleichzeitig ist eine hohe Formstabilität des Beatmungsschlauchs 10 auf eine einfache Art und Weise gegeben.

## Beschreibung

Die Erfindung betrifft einen beheizbaren Beatmungsschlauch und ein Verfahren zur Herstellung des Beatmungsschlauchs, bestehend aus einem Schlauch aus einem elastischen Material und einer daran angebrachten, in Längsrichtung des Schlauchs verlaufenden Heizwendel aus einem elektrisch leitfähigen Material.

Ein derartiger beheizbarer Beatmungsschlauch ist durch das Deutsche Gebrauchsmuster G 9200567.5 bekanntgeworden.

Wenn ein Patient von sich aus nicht in der Lage ist, ausreichend zu atmen, d. h., für eine genügende Sauerstoffaufnahme und Kohlendioxidabgabe zu sorgen, ist eine künstliche Beatmung notwendig. Eine künstliche Beatmung wird auch meist in Zusammenhang mit Narkosen angewandt.

Bei einer künstlichen Beatmung werden die Beatmungsgase in der Regel über Beatmungsschläuche vom Beatmungsgerät an den Patienten weitergeleitet. Diese Schläuche können beheizbar sein. Dabei muß darauf geachtet werden, daß der Beatmungsschlauch aus einem Material gefertigt ist, das allergische bzw. überempfindliche Reaktionen beim Patienten als auch bei dem behandelnden Arzt oder dem Pflegepersonal vermeidet.

Beatmungsschläuche sind vor allem in der Unfallmedizin im Einsatz, damit sie insbesondere in Notfällen Patienten mit Beatmungsluft versorgen können. Zur besseren Kontrolle der Luftzufuhr und der Handhabung durch das Pflegepersonal können Beatmungsschläuche sichtdurchlässig ausgebildet sein.

Die dem Patienten zugeführte Beatmungsluft sollte, weil sie nicht wie unter natürlichen Verhältnissen in der Nase des Pataienten klimatisiert, auf die Körpertemperatur des Patienten erwärmt werden.

Da Beatmungsschläuche an den verschiedensten Notfallorten eingesetzt werden, müssen sie auch bei den unterschiedlichen Rahmenbedingungen, den an einem Unfallort vorherrschenden Witterungsbedingungen, verwendet werden können. Beispielsweise führen niedrige Außentemperaturen oder schon die grundsätzliche Temperaturdifferenz zwischen der Umgebungstemperatur und der Inspirations- bzw. Expirationsluft dazu, daß nicht beheizte Beatmungsschläuche an ihrer Innenumfangsfläche beschlagen, d.h. Feuchtigkeit auskondensiert. Um dies zu vermeiden, ist der bekannte Beatmungsschlauch beheizbar ausgebildet.

Der bekannte Beatmungsschlauch weist eine Heizwendel auf, die auf der Außenumfangsfläche des Schlauchs angebracht ist. Damit nun die zugeführte Beatmungsluft innerhalb des Beatmungsschlauchs beheizt wird, muß der gesamte Schlauch über die Heizwendel beheizt werden, damit die Heizwärme über den Schlauch auf die Beatmungsluft übertragen werden kann. Nachteiligerweise ist eine große Heizleistung notwendig, da die Beatmungsluft nur indirekt über die Schlauchwand beheizt wird. Dieser Erwärmungsprozeß der Beatmungsluft wird noch dadurch erschwert, daß der Schlauch nur aus Materialien hergestellt werden kann, die zwar hautverträglich sind und keine allergischen Reaktionen auslösen können, aber meist schlechte Wärmeleiter sind.

Aus der GB 2173274 A ist ebenfalls ein beheizbarer Beatmungsschlauch bekannt, der einen Schlauch aufweist, der sowohl von einer Heizwendel als auch von einer Stützwendel umgeben ist. Beim Heizvorgang wird auch Heizwärme auf die Stützwendel übertragen, so daß die erzeugte Heizenergie nicht nur bestimmungsgemäß eingesetzt wird. Durch die Ausbildung einer Heizwendel und einer Stützwendel ist der bekannte Beatmungsschlauch außerdem aufwendig und teuer herzustellen.

Weiterhin beschreibt auch die US 4,967,744 einen beheizbaren Beatmungsschlauch. Dieser Beatmungsschlauch umfaßt einen äußeren Schlauch, in dem ein innerer Schlauch verschieblich ist. Die Formstabilität ergibt sich durch eine Stützwendel auf der Außenumfangsfläche des äußeren Schlauchs, während die Erwärmung der Beatmungsluft durch eine Heizwendel auf der Außenumfangsfläche des inneren Schlauchs durchgeführt wird. Folglich ist auch dieser Beatmungsschlauch ähnlich wie die schon erörterten Beatmungsschläuche aufgebaut.

Der vorliegenden Erfindung liegt daher die Aufgabe zugrunde, den bekannten Beatmungsschlauch derart weiterzuentwickeln und ein Verfahren zu seiner Herstellung anzubieten, daß die durch die Heizwendel erzeugte Wärme effektiver auf die den Beatmungsschlauch durchströmende Beatmungsluft übertragen wird und gleichzeitig eine hohe Formstabilität des Beatmungsschlauchs auf eine einfache Art und Weise gegeben ist.

Diese Aufgabe wird dadurch gelöst, daß die Außenumfangsfläche der Heizwendel an einer Innenoberfläche im Lumen des Schlauchs befestigt ist.

Durch das Anbringen der Heizwendel im Lumen des Beatmungsschlauchs wird erreicht, daß die Beatmungsluft direkt mit der Heizwendel, die nur eine Isolationsschutzschicht aufweist, in Kontakt kommen kann. Die Kondensation von vernebelten Flüssigkeiten wird innerhalb des Beatmungsschlauchs verhindert.

Als elektrisch leitfähige Materialien für die Heizwendel können alle denkbaren leitfähigen Materialen verwendet werden. Zur besseren Wärmeübertragung ist es allerdings bevorzugt, daß die Heizwendel aus einer dünnen Kupferlitze oder Metallitze gebildet ist. Die Kupferlitze kann auf die Innenoberfläche des Schlauches aufgeklebt oder aufgeschweißt oder auf andere Art und Weise dauerhaft befestigt werden.

Durch den erfindungsgemäßen Beatmungsschlauch läßt sich erreichen, daß die Beatmungsluft am patientenseitigen Schlauchende adäquat temperiert wird. Durch die direkte Wärmeübertragung auf die Beatmungsluft wird eine Kondensation der feuchten Bestandteile der Beatmungsluft an der Innenoberfläche des Schlauchs ebenso ausgeschlossen, wie eine Unterkühlung des Patienten. Auf Grund dieser vorteilhaften und effektiven Wärmeübertragung von der Heizwendel auf die Beatmungsluft kann die aufgenommene elektrische Leistung gering gehalten werden, was insbesondere bei Batteriebetrieb vorteilhaft ist.

Zum Schutz der Heizwendel und auch zur Vermeidung von elektrischen Kurzschlüssen ist es bevorzugt, daß die Innenumfangsfläche der Heizwendel mit einem Schutzfilm überzogen ist. Als Schutzfilm ist beispielsweise eine Siliconummantelung geeignet. Durch den Schutzfilm kann auch erreicht werden, daß die Innenoberfläche des erfindungsgemäßen Beatmungsschlauchs geglättet und die Zwischenräume zwischen den einzelnen Wendelabschnitten der Heizwendel ausgeglichen werden.

Bei einer besonders bevorzugten Ausführungsform ist die Heizwendel als Doppelwendel ausgebildet. Dies hat den Vorteil, daß durch die an der Innenoberfläche des Beatmungsschlauchs angebrachten Heizdrähte einerseits eine verstärkte Armierung zur Stabilitätserhöhung und andererseits eine Ausbildung zur Erwärmung des Beatmungsschlauchs gegeben ist.

Bei einer Weiterbildung dieser Ausführungsform sind die einen stromleitenden Enden der Doppelwendel auf eine elektrisch leitfähige Weise miteinander verbunden, und die anderen Enden sind an einen Heizstromgenerator anschließbar. Aus diesem Grund muß der erfindungsgemäße Beatmungsschlauch nur einenends mit dem Heizstromgenerator konnektiert werden, während das andere Ende ohne Anschluß bleibt. Der bekannte Beatmungsschlauch weist dagegen an seinen beiden Enden elektrische Anschlüsse auf, über die die Heizwendel mit einem Heizstromgenerator verbunden wird.

Bevorzugt sind die einzelnen Wendeln der Doppelwendel aus unterschiedlichen Materialien. Einerseits kann dadurch die elektrische Leitfähigkeit gezielt beeinflußt werden und andererseits kann die Formstabilität des Beatmungschlauches erweitert beeinflußt werden.

Zur weiteren Stabilitätserhöhung insbesondere zur Erhöhung der Knickstabilität ist zwischen der Doppelwendel ein Vollmaterialstreifen angeordnet. Der Vollmaterialstreifen könnte auch in unterschiedlichen Farben an dem Schlauch angebracht sein, um verschiedene Beatmungsschläuche zu kennzeichnen, d. h., um sie voneinander unterscheiden zu können. Vorteilhafterweise kann der Vollmaterialstreifen auch als Abstandshalter zwischen den einzelnen Wendelabschnitten der Heizwendel dienen.

Bevorzugt ist es, daß der Vollmaterialstreifen aus Silicon besteht. Dies trägt zusätzlich zur Herstellung des beheizbaren Beatmungsschlauchs aus hautverträglichem Material bei.

Bei einer anderen Variante ist der Schlauch aus einem Material mit einer geringen Wärmeleitfähigkeit und der Schutzfilm aus einem Material mit einer hohen Wärmeleitfähigkeit gefertigt. Ein derartiger Beatmungsschlauch genügt den o. g. Anforderungen an einen Beatmungsschlauch in nahezu optimaler Weise, da einerseits nach außen möglichst wenig Wärme abgegeben wird, und andererseits eine gute nach innen gerichtete Wärmeabgabe gegeben ist. Aus diesem Grund kann der Heizvorgang des Beatmungsschlauchs mit geringem Energieaufwand durchgeführt werden.

Bei einer weiteren Ausführungsform ist der Schlauch aus einem Nitrilkautschuk oder einem Siliconelastomer gefertigt. Ein Beatmungsschlauch aus Nitrilkautschuk weist eine permeationsstabile Schlauchwandung auf. Dieses Material ist weitgehend narkosegasundurchlässig und daher bevorzugt für einen im Narkosebereich eingesetzten Beatmungsschlauch geeignet. Der erfindungsgemäße Beatmungsschlauch könnte aber auch aus anderen Kunststoffen, insbesondere Thermoplasten und Elastomeren hergestellt werden. Bevorzugt sind gummielastische Materialien, die einerseits eine Formstabilität und andererseits eine Biegung des Beatmungsschlauchs garantieren, ohne daß das Lumen kollabiert oder abgeknickt wird.

In den Rahmen der vorliegenden Erfindung fällt auch ein Verfahren zur Herstellung eines beheizbaren Beatmungsschlauchs, bei dem in einem ersten Verfahrensschritt ein Schutzfilm (vorzugsweise eine Isolierschicht) derart auf einen später entfernbaren Formstab aufgezogen wird, daß dieser eine dünne Umhüllung des Formstabes bildet; in einem zweiten Verfahrensschritt wird eine Heizwendel auf den Schutzfilm aufgebracht, und anschließend wird eine die Heizwendel und den Schutzfilm umgebende zylindrische Schlauchwand aus einem formstabilen, elastischen Material aufgetragen, die eine materialschlüssige Verbindung mit Teilen des Schutzfilms unter Bildung einer einheitlichen Materialwandung eingeht. Der Formstab kann jede beliebige Querschnittsform haben, so daß Beatmungsschläuche mit kreisförmigem Querschnitt oder anderen Querschnittsformen erzeugt werden können.

Durch dieses Verfahren wird die Heizwendel vollständig in den Schlauch integriert, wobei die Heizwendel vorteilhafterweise nach außen hin wärmeisoliert ist und zum Lumen hin die erzeugte Wärme gut abgeben kann. Ein durch das erfindungsgemäße Verfahren hergestellter Beatmungsschlauch besitzt auch eine ausreichende Formstabilität, die ihn nahezu universell einsetzbar macht.

Bei einer Weiterentwicklung des Verfahrens wird der Schutzfilm durch Aufziehen eines Siliconbandes erzeugt und zur Ausbildung der Schlauchwand ein vulkanisierbares oder vernetzbares Kautschukmaterial verwendet. Dies ermöglicht, die materialschlüssige Verbindung zwischen der Schlauchwand und dem Schutzfilm. Die materialschlüssige Verbindung kann durch eine Vernetzung der Moleküle der verwendeten Kunststoffe unterstützt werden, beispielsweise durch eine additionsvernetzte oder peroxydische Vernetzung.

Weitere Vorteile ergeben sich aus der Beschreibung und der beigefügten Zeichnung. Ebenso können die vorstehend genannten und hier noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand eines Ausführungsbeispieles näher erläutert. Es zeigt:
- Fig. 1: eine perspektivische Darstellung des erfindungsgemäßen Beatmungsschlauchs mit teilweise aufgerissenem Schlauch zur Ansicht der Heizwendel;
- Fig. 2: eine schematische Schnittdarstellung des erfindungsgemäßen Beatmungsschlauchs nach Fig. 1.

Die einzelnen Figuren der Zeichnung zeigen den erfindungsgemäßen Gegenstand teilweise stark schematisiert und sind nicht notwendigerweise maßstäblich zu verstehen.

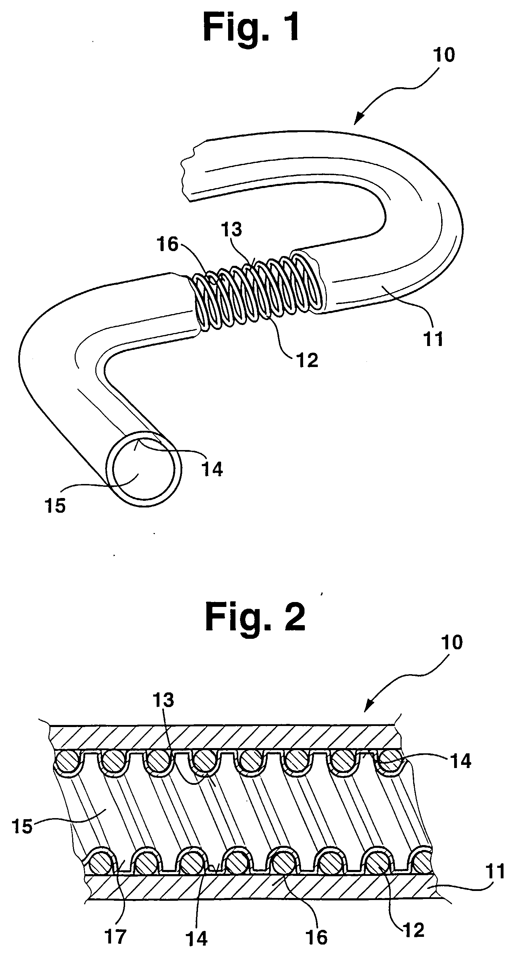

In der Fig. 1 ist ein Beatmungsschlauch 10 perspektivisch dargestellt, der im wesentlichen aus einem Schlauch 11 und einer Heizwendel 12 besteht. In der Figur ist der Schlauch 11 im aufgerissenen Zustand gezeigt, um den Blick auf die Heizwendel 12 freizugeben. Der Schlauch 11 ist aus einem formstabilen, elastischen Material gefertigt, um unterschiedliche auf den jeweiligen Einsatz angepaßte Formen annehmen zu können. Die Heizwendel 12 verläuft in Längsrichtung des Schlauchs 11 und ist mit einer Außenumfangsfläche 13 an einer Innenoberfläche 14 im Lumen 15 des Schlauchs 11 befestigt. Die Außenumfangsfläche 13 der Heizwendel 12 kann an der Innenoberfläche 14 des Schlauchs 11 angeklebt, angeschweißt oder auf eine andere Art und Weise befestigt sein. Es können sämtliche Befestigungsarten verwendet werden, die eine dauerhafte Fixierung einer elektrisch leitfähigen, vorzugsweise aus Kupfer oder einem anderen Metall bestehenden Heizwendel 12 an einem Schlauch 11 aus einem hautverträglichen Kunststoff ermöglichen. Zum Schutz der Innenumfangsfläche 16 der Heizwendel 12 kann die in der Regel mit einer Isolationsschicht überzogene Heizwendel 12 zusätzlich mit einem in der Fig. 1 nicht sichtbaren Schutzfilm überzogen sein, wie dies beispielhaft Fig. 2 zeigt. Der Schutzfilm gewährleistet zusätzlich eine dauerhafte Fixierung der Heizwendel 12 an der Innenoberfläche 14, weil der Schutzfilm auch eine materialschlüssige Verbindung mit der Innenoberfläche 14 eingehen kann. Dies kann beispielsweise durch Einvulkanisieren oder Vernetzen der verwendeten Kunststoffe erfolgen. Der Schutzfilm bildet eine innige Verbindung mit der Innenoberfläche 14. Der Schutzfilm sorgt auch dafür, daß die Zwischenräume zwischen den einzelnen Wendelabschnitten der Heizwendel 12 ausgeglichen und eine glatte Oberfläche im Lumen 15 erzeugt wird. Zur optimalen, nahezu verlustfreien Wärmeübetragung von der Heizwendel 12 auf die Beatmungsluft kann der Schutzfilm aber auch weggelassen werden. Ist dies der Fall, muß die Heizwendel 12 unabdingbar mit einer Isolationsfilmschicht überzogen sein.

Da der Schlauch aus einem Material mit einer geringen Wärmeleitfähigkeit gefertigt ist und das Material des Schutzfilms eine hohe Wärmeleitfähigkeit aufweist, kann die im Innern des Beatmungsschlauchs 10 durchströmende Beatmungsluft mit einem geringen Energieaufwand erwärmt werden. Zwischen den einzelnen Wendelabschnitten der Heizwendel 12 kann es zu Strömungswirbeln kommen, die eine Erwärmung der Beatmungsluft unterstützen und eine Kondensation aus feuchtigkeitsbeladenem Gas verhindern.

In Fig. 2 ist ein Längsschnitt durch den Beatmungsschlauch 10 gezeigt. Im Lumen 15 des Beatmungsschlauchs 11 ist eine Heizwendel 12 zur Erwärmung der zum Patienten geleiteten Beatmungsluft angebracht. Die Heizwendel 12 ist mit der Außenumfangsfläche 16 an der Innenoberfläche 14 durch ein Schweißen oder Ankleben befestigt. Die Innenumfangsfläche 13 der Heizwendel 12 ist mit einem Schutzfilm 17 überzogen, der die Zwischenräume zwischen den einzelnen Wendelabschnitten der Heizwendel 12 überbrückt und die Heizwendel 12 zum Lumen 15 abschirmt. Der Schutzfilm 17 ist bei der gezeigten Ausführungsform zwischen den einzelnen Wendelabschnitten der Heizwendel 12 zumindest teilweise bis zur Innenoberfläche 14 heruntergezogen, um eine dauerhafte Verbindung mit dem Schlauch 11 einzugehen. Dies gewährleistet eine zusätzliche örtliche Fixierung der Heizwendel 12.

In einer in den Figuren nicht dargestellten Ausführungsform könnte die Heizwendel 12 auch als Doppelwendel ausgebildet sein, bei der einenends die Enden auf eine elektrisch leitfähige Weise miteinander verbunden sind und die anderenends mit Enden ausgebildet ist, die aus dem Beatmungsschlauch 10 herausgeführt sind, um sie mit einem Heizstromgenerator verbinden zu können. Die einzelnen Wendeln können aus unterschiedlichen elektrisch leitfähigen Materialen bestehen, damit die geforderte Formstabilität des Beatmungsschlauches 10 erhalten bleibt. Zur Erweiterung der Einsatzmöglichkeiten des Beatmungsschlauchs 10 können die verschiedensten Konnektiereinrichtungen an dem Beatmungsschlauch vorgesehen sein, wie beispielsweise Luer-Lockverbindungen oder dergleichen.

Ein beheizbarer Beatmungsschlauch 10 besteht aus einem Schlauch 11 aus einem elastischen Material und einer daran angebrachten, in Längsrichtung des Schlauchs 11 verlaufenden Heizwendel 12 aus einem elektrisch leitfähigen Material. Die Außenumfangsfläche 13 der Heizwendel 12 ist an einer Innenoberfläche 14 im Lumen 15 des Schlauchs 11 befestigt. Daher kann die durch die Heizwendel 12 erzeugte Wärme effektiver auf die den Beatmungsschlauch 10 durchströmende Beatmungsluft übertragen werden. Gleichzeitig ist eine hohe Formstabilität des Beatmungsschlauchs 10 auf eine einfache Art und Weise gegeben. Das Lumen 15 kann sich aufgrund der Heizwendel 12 auch bei gekrümmtem Beatmungsschlauch 10 nicht verschließen. Die als Armierung wirkende Heizwendel 12 kann durch zusätzliche Bänder oder Materialeinlagerungen im unmittelbaren Bereich der Innenoberfläche 14 des Schlauches 11 noch verbessert werden. Die Innenoberfläche 14 kann auch eben und glatt ausgebildet sein.

## Patentansprüche

1. Beheizbarer Beatmungsschlauch (10), bestehend aus einem Schlauch (11) aus einem elastischen Material und einer daran angebrachten, in Längsrichtung des Schlauchs (11) verlaufenden Heizwendel (12) aus einem elektrisch leitfähigen Material,
dadurch gekennzeichnet,
daß die Außenumfangsfläche (13) der Heizwendel (12) an einer Innenoberfläche (14) im Lumen (15) des Schlauchs (11) befestigt ist.

2. Beatmungsschlauch nach Anspruch 1, dadurch gekennzeichnet, daß die Innenumfangsfläche (16) der Heizwendel (12) mit einem Schutzfilm (17) überzogen ist.

3. Beatmungsschlauch nach Anspruch 1 oder 2, dadurch gekennzeichnet, daß der Schutzfilm (17) zwischen einzelnen Wendelabschnitten der Heizwendel (12) materialschlüssig mit der Innenoberfläche (14) des Schlauchs (11) verbunden ist.

4. Beatmungsschlauch nach einem der vorhergehenden Ansprüche, dadurch gekennzeichnet, daß die Heizwendel (12) als Doppelwendel ausgebildet ist.

5. Beatmungsschlauch nach Anspruch 4, dadurch gekennzeichnet, daß die Doppelwendel aus Wendeln hergestellt ist, die aus einem unterschiedlichen Material bestehen.

6. Beatmungsschlauch nach Anspruch 4 oder 5, dadurch gekennzeichnet, daß einenends die stromleitenden Enden der Doppelwendel auf eine elektrisch leitfähige Weise miteinander verbunden sind und daß anderenends die Enden der Doppelwendel an einen Heizstromgenerator anschließbar sind.

7. Beatmungsschlauch nach Anspruch 4, 5 oder 6, dadurch gekennzeichnet, daß zwischen der Doppelwendel ein Vollmaterialstreifen angeordnet ist.

8. Beatmungsschlauch nach Anspruch 7, dadurch gekennzeichnet, daß der Vollmaterialstreifen aus Silicon besteht.

9. Beatmungsschlauch nach einem der vorhergehenden Ansprüche, dadurch gekennzeichnet, daß der Schlauch (11) aus einem Material mit einer geringen Wärmeleitfähigkeit und der Schutzfilm (17) aus einem Material mit einer hohen Wärmeleitfähigkeit gefertigt ist.

10. Beatmungsschlauch nach einem der vorhergehenden Ansprüche, dadurch gekennzeichnet, daß der Schlauch (11) aus einem Nitrilkautschuk oder einem Siliconelastomer gefertigt ist.

11. Verfahren zur Herstellung eines beheizbaren Beatmungsschlauchs, bei dem
in einem ersten Verfahrensschritt ein Schutzfilm derart auf einen später entfernbaren Formstab aufgezogen wird, daß dieser eine dünne, Umhüllung des Formstabes bildet,
in einem zweiten Verfahrensschritt eine Heizwendel (12) auf den Schutzfilm (17) aufgebracht wird,
und anschließend eine die Heizwendel (12) und den Schutzfilm (17) umgebende zylindrische Schlauchwand aus einem formstabilen, elastischen Material aufgetragen wird, die eine materialschlüssige Verbindung mit Teilen des Schutzfilms (17) unter Bildung einer einheitlichen Materialwandung eingeht.

12. Verfahren nach Anspruch 11, dadurch gekennzeichnet, daß der Schutzfilm (17) durch Aufziehen eines Siliconbands erzeugt und zur Ausbildung der Schlauchwand ein vulkanisierbares oder vernetzbares Kautschukmaterial verwendet wird.
